# EUROPÄISCHE PATENTSCHRIFT

(11) **EP 0 378 619 B1**
(45) Veröffentlichungstag und Bekanntmachung des Hinweises auf die Patenterteilung: **24.03.1993**
(21) Anmeldenummer: 89906696.3
(22) Anmeldetag: 23.06.1989
(51) Int. Cl.: A61F 2/36

(54) **SELBSTBLOCKIERENDER SCHAFTTEIL**
SELF-BLOCKING STEM
TIGE AUTOBLOQUANTE

(30) Priorität: 05.07.1988 CH 2559/88
(43) Veröffentlichungstag der Anmeldung: 25.07.1990
(73) Patentinhaber: AO-FORSCHUNGSINSTITUT DAVOS, 7270 Davos-Platz (CH)
(72) Erfinder: KLAUE, Kaj, F-75017 Paris (FR)
(74) Vertreter: Lusuardi, Werther Giovanni, Dr.
(86) Internationale Anmeldenummer: CH8900122
(87) Internationale Veröffentlichungsnummer: WO9000374

(56) Entgegenhaltungen:
- EP-A- 0 061 993
- EP-A- 0 197 441
- WO-A-87/04916
- DE-A- 1 810 799
- DE-A- 2 842 847
- DE-A- 2 948 792
- DE-A- 3 003 758
- DE-A- 3 536 894
- FR-A- 2 104 009
- FR-A- 2 438 468
- FR-A- 2 519 248

## Beschreibung

Die Erfindung bezieht sich auf ein, in einen Röhrenknochen implantierbares Schaftteil gemäss dem Oberbegriff von Anspruch 1.

Es sind eine grosse Zahl von Schaftteilen für zementlos zu implantierenden Gelenkendoprothesen bekannt, welche den Anspruch erheben eine automatische Blockierung des Schaftes in der Markhöhle zu bewirken.

Beispielsweise ist aus der WO 86/06954 ein Hüftprothesenschaft bekannt mit je einem medialen und lateralen durch Verbindungsstrukturen miteinander verbundene Schenkel, der sich bei Biegebeanspruchung im Querschnitt vergrössert und damit den Schaft gegen die Markhöhle fixiert. Der Kontakt zum Knochen bleibt bei diesem Schaft gemäss dem Stand der Technik immer auf 3 Punkte beschränkt. Ein punktueller Kontakt bewirkt jedoch hohe lokale Belastungen.

Weiter ist aus der EP-A1-0 061 993 ein mit einem Matrix-Kunststoff benetztes Fasergelege als Schaftteil für eine Gelenkprothese bekannt geworden, welcher anlässlich seiner Implantation ausgehärtet wird. Um diesen bekannten Schaft zu implantieren benötigt man aufwendige Hilfsmittel, um das mit Monomer benetzte Fasergelege an die Markhöhle anzupassen und auszuhärten. Besonders nachteilig ist die durch die Aushärtung bewirkte Immobilisierung der Fasern, so dass jegliche räumliche Adaptationsfähigkeit des Schaftes verloren geht.

Hier will die Erfindung Abhilfe schaffen. Der Erfindung liegt die Aufgabe zugrunde, einen selbstblockierenden Schaftteil für eine Gelenkendoprothese zu schaffen, dessen Oberfläche dank seiner Adaptabilität zu flächenhaften Kontakten mit dem Knochen fähig ist, so dass möglichst geringe lokale Belastungen auftreten.

Die Erfindung löst die gestellte Aufgabe mit einem Schaftteil, welches die Merkmale des Anspruchs 1 aufweist.

Die durch die Erfindung erreichten Vorteile sind im wesentlichen darin zu sehen, dass dank des erfindungsgemässen Schaftteils eine maximale Adaptationsfähigkeit der Schaftoberfläche an die Anatomie der Markhöhle erreicht wird und dass sowohl die Implantation als auch die Entfernung des Schaftteils rasch und ohne Komplikationen möglich ist.
Durch die vorzugsweise offene, kegelstumpfförmige Konstruktion des Schaftes bleibt dabei im Gegensatz zu herkömmlichen Systemen die Markhöhle offen, so dass der Knochen weiterhin durchblutet wird und der Knochenumbau bestehen bleibt.
Ein weiterer Vorteil besteht schliesslich darin, dass wegen des im wesentlichen kreissymmetrisch oder ellipsenförmïg ausgebildeten Schaftquerschnittes, allfällige Rotationskräfte, wie sie vor allem bei Hüftprothesen in rascher intermittierender Weise auftreten, wesentlich besser aufgenommen und abgeleitet werden können, im Vergleich zu den meist blattförmig ausgebildeten Schäften gemäss dem Stand der Technik.

Ein Ausführungsbeispiel der Erfindung, welches zugleich das Funktionsprinzip erläutert, ist in der Zeichnung dargestellt und wird im folgenden näher beschrieben.
Fig. 1 stellt eine perspektivische Ansicht des als Hüftprothesen-Femurteil ausgebildeten Schaftes von lateral dar;
Fig. 2 stellt einen Querschnitt längs der Linie II-II durch den Schaft gemäss Fig. 1 dar.
Fig. 3 stellt einen Querschnitt gemäss Fig. 2 durch einen längsgeschlitzten erfindungsgemässen Schaftteil dar.
Fig. 4 stellt eine perspektivische Ansicht des noch nicht verklemmten erfindungsgemässen Schaftteils dar.
Fig. 5 stellt eine perspektivische Ansicht des verklemmten erfindungsgemässen Schaftteils dar.

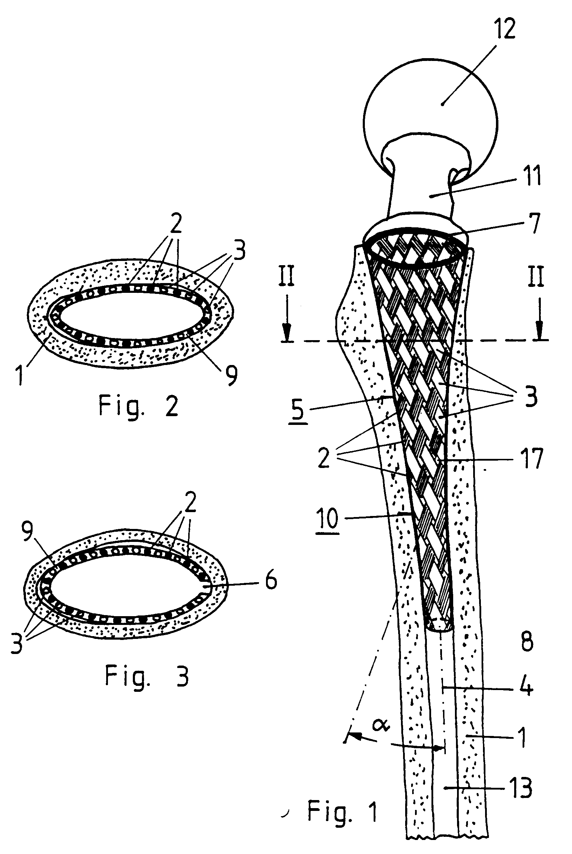

Wie in Fig. 1 dargestellt besteht der als Hüftprothesen-Femurteil ausgebildete, erfindungsgemässe Schaft im wesentlichen aus einem kegelstumpfförmigen von proximal nach distal im Einklang mit der Anatomie der Markhöhle sich verengenden Geflecht 5, welches aus zwei gegeneinander gekreuzten Serien von Fasern 2,3 besteht. Vorzugsweise weist das kegelstumpfförmige Geflecht 5 im distalen Bereich einen annähernd kreisförmigen Querschnitt und im proximalen Bereich einen annähernd elliptischen Querschnitt auf. Wie in Fig. 1 angedeutet sind die Aussenkonturen des Geflechtes 5 im Längsschnitt konkav ausgebildet, d.h. der Querschnitt ist in der Mitte gegenüber einem regelmässigen Kegelstumpf mit geradlinigen Aussenkonturen verengt. Die Fasern 2,3 bestehen aus einem körperverträglichen Metall, bzw. einer dazu geeigneten Metallegierung oder einem Kunststoffmaterial. Bevorzugt werden Kohlenstoffasern, beispielsweise aus Pyrocarbon. Die Fasern 2,3 bilden dabei einen Winkel α mit der Längsachse 4 des durch das Geflecht 5 gebildeten hohlen Kegelstumpfes. In der Regel ist der Winkel α für beide Serien von Fasern 2,3 annähernd gleich gross, doch kann wegen der besseren Abstützung des Geflechtes 5 im proximalen Femurteil der Winkel α längs einer Faser 2, bzw. 3 auch variabel sein und vorzugsweise von distal nach proximal in seinem Werte abnehmen.
Bei einer bevorzugten Ausführungsform sind die Zwischenräume 17 des kegelstumpfförmigen Geflechts 5 aus Fasern 2 und 3, wie in Fig. 2 dargestellt, mit einem elastomeren Material 9, beispielsweise mit Silikonkautschuk oder Silastik ausgefüllt, um eine glatte Oberfläche des Schaftes zu erzeugen. Dieser Umstand ist deshalb von Bedeutung, weil es sich gezeigt hat, dass in die grobstrukturierte Oberfläche des nackten Geflechtes 5 neugebildetes Knochenmaterïal 1 sehr leicht einwachsen kann, was die Entfernbarkeit des Schaftes bei einer möglichen Reoperation erheblich erschwert.

Wie in Fig. 3 gezeigt, kann das kegelstumpfförmige Geflecht 5 bei einer weiteren bevorzugten Ausführungsform der Erfindung einen axial verlaufenden Schlitz 6 aufweisen.

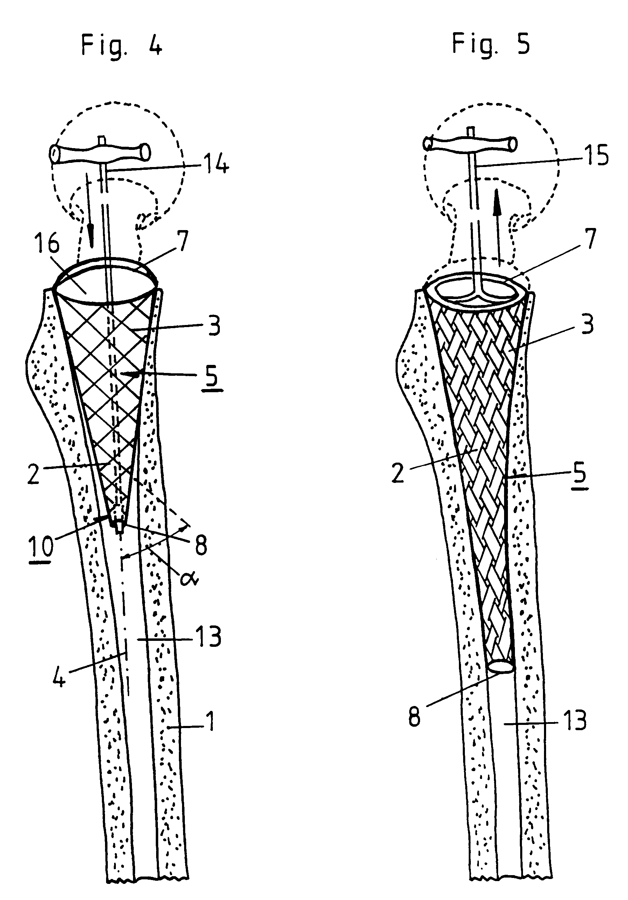

Das kegelstumpfförmige Geflecht 5 weist im proximalen Bereich eine ringförmige Struktur 7 auf, an der die proximalen Enden der Faserserien 2 und 3 befestigt sind und im distalen Bereich eine ringförmige Struktur 8, an der die distalen Enden der Faserserien 2 und 3 befestigt sind. Diese ringförmigen Strukturen 7,8 , welche kreisförmig oder elliptisch ausgebildet sein können, ermöglichen es durch Ausübung einer Zugkraft auf einen der beiden Ringe bei gleichzeitiger Fixierung des anderen Ringes die Geometrie des Geflechtes 5, bzw. des dadurch gebildeten hohlen Kegelstumpfes zu verändern, insbesondere Länge und Durchmesser des Schaftes zu variieren. Diese Fähigkeit des erfindungsgemässen Schaftteiles findet bei der Implantation, bzw. Entfernung der damit ausgestatteten Prothese ihre Anwendung und wird nachstehend anhand der Fig. 4 und 5 im Einzelnen beschrieben.

Die Implantation des erfindungsgemässen Schaftteiles erfolgt in üblicher Weise durch Einführung des Schaftes 10 in die Markhöhle 13 des Femur 1. Sobald der Schaft 10 eine erste provisorische Verkeilung in der Markhöhle 13 gefunden hat wird, wie in Fig. 4 dargestellt, mittels eines geeigneten langstieligen Instrumentes 14, die distale ringförmige Struktur 8 erfasst und durch Druckanwendung weiter nach distal geschoben. Bei diesem Vorgang wird auf die Fasern 2,3, welche mit ihren proximalen Enden an der in der Markhöhle 13 fixierten, ringförmigen Struktur 7 befestigt sind, eine Zugkraft augeübt, welche zu einer Verlängerung des hohlen kegelstumpfförmigen Geflechts 5 bei gleichzeitiger Verjüngung führt. Der Winkel α zwischen den Fasern 2, bzw. 3 und der Längsachse 4 des durch das Geflecht 5 gebildeten hohlen Kegelstumpfes nimmt dabei in seinem Werte ab.
Durch diese Streckung des kegelstumpfförmigen Geflechtes 5 kann ein optimaler Kontakt mit der Markhöhle 13 gefunden werden, wie in Fig. 1 dargestellt. Diese flächenförmige Verankerung bleibt auch nach der Implantation erhalten, weil jedes mögliche "Einsinken" des Schaftes zu einer Aufweitung des kegelstumpfförmigen Geflechtes 5 führen muss.
Der Einsatz des Implantationsinstrumentes 14 gestaltet sich im übrigen äusserst einfach, da das kegelstumpfförmige Geflecht 5 durchgehend offen ausgebildet ist, so dass die Spitze des Implantationsinstrumentes 14 direkt durch den Innenraum 16 des kegelstumpfförmigen Geflechts 5 mit der ringförmigen Struktur 8 in Eingriff gebracht werden kann.

Im Gegensatz zu zementierten oder strukturierten, zementlos implantierten Schäften gemäss dem Stand der Technik, gestaltet sich die Entfernung des erfindungsgemässen Schaftteils überaus einfach. Es genügt mittels eines geeigneten Instrumentes 15, die proximale ringförmige Struktur 7 zu erfassen und weiter nach proximal zu ziehen. Bei diesem Vorgang wird auf die Fasern 2,3, welche mit ihren distalen Enden an der in der Markhöhle 13 fixierten, ringförmigen Struktur 8 befestigt sind, eine Zugkraft ausgeübt, welche zu einer Verlängerung des hohlen kegelstumpfförmigen Geflechts 5 bei gleichzeitiger Verjüngung desselben führt, so dass der Schaftteil sich von der Markhöhle 13 löst und ohne Mühe daraus entfernt werden kann.

## Patentansprüche

1. In einen Röhrenknochen (1) implantierbares Schaftteil für eine Gelenkendoprothese oder als Knochenschaftersatz, das im wesentlichen ein hohles, in Länge und Durchmesser variables, kegelstumpfförmiges Geflecht (5) umfasst, welches aus zwei gegeneinander gekreuzten Serien von Fasern (2,3) besteht, dadurch gekennzeichnet, dass das kegelstumpfförmige Geflecht (5)
A) im proximalen Bereich eine ringförmige Struktur (7) aufweist, an der die proximalen Enden der Fasern (2;3) befestigt sind und
B) im distalen Bereich eine ringförmige Struktur (8) aufweist, an der die distalen Enden der Fasern (2;3) befestigt sind.

2. Schaftteil nach Anspruch 1, dadurch gekennzeichnet, dass die Fasern einen Winkel α mit der Längsachse (4) des durch das Geflecht (2,3) gebildeten Kegelstumpfes einschliessen.

3. Schaftteil nach Anspruch 2, dadurch gekennzeichnet, dass der Winkel α für beide Serien von Fasern (2,3) annähernd gleich gross ist.

4. Schaftteil nach einem der Ansprüche 1 bis 3, dadurch gekennzeichnet, dass der Winkel α längs der Faser (2;3) variabel ist und vorzugsweise von distal nach proximal abnimmt.

5. Schaftteil nach einem der Ansprüche 1 bis 4, dadurch gekennzeichnet, dass die Zwischenräume (17) des kegelstumpfförmigen Geflechtes (5) mit einem elastomeren Material (9), vorzugsweise mit Silikonkautschuk oder Silastik gefüllt sind.

6. Schaftteil nach einem der Ansprüche 1 bis 5, dadurch gekennzeichnet, dass das kegelstumpfförmige Geflecht (5) einen axial verlaufenden Schlitz (6) aufweist.

7. Schaftteil nach einem der Ansprüche 1 bis 6, dadurch gekennzeichnet, dass die Fasern (2;3) Kohlenstoffasern, vorzugsweise aus Pyrocarbon sind.

8. Schaftteil nach einem der Ansprüche 1 bis 7, dadurch gekennzeichnet, dass die ringförmige Struktur (7) durchgehend offen ausgebildet ist.

9. Schaftteil nach einem der Ansprüche 1 bis 8, dadurch gekennzeichnet, dass das kegelstumpfförmiges Geflecht (5) im distalen Bereich einen annähernd kreisförmigen querschnitt und im proximalen Bereich einen annähernd elliptischen Querschnitt aufweist.

10. Schaftteil nach einem der Ansprüche 1 bis 9, dadurch gekennzeichnet, dass die Aussenkonturen des Geflechtes (5) im Längsschnitt konkav ausgebildet sind.

## Claims

1. A shaft part for a joint endoprosthesis or a bone-shaft replacement, implantable into a tubular bone (1), which essentially comprises a hollow, frusto-conical braid (5), variable in length and diameter, which consists of two mutually crossing series of fibers (2,3), characterized in that the frusto-conical braid (5) comprises
A) an annular structure (7) in the proximal zone to which the proximal ends of the fibers (2,3) are fastened; and
B) an annular structure (8) in the distal zone to which the distal ends of the fibers (2,3) are fastened.

2. Shaft part according to claim 1, characterized in that the fibers subtend an angle α with the longitudinal axis (4) of the frustum of a cone formed by the braid (2,3).

3. Shaft part according to claim 2, characterized in that the angle α is approximately the same for both series of fibers (2, 3).

4. Shaft part according to one of the claims 1 to 3, characterized in that the angle α along the fibers (2,3) varies and preferably decreases from the distal to the proximal zone.

5. Shaft part according to one of the claims 1 to 4, characterized in that the gaps (17) in the frusto-conical braid (5) are filled with an elastomer (9), preferably with silicone rubber or Silastic.

6. Shaft part according to one of the claims 1 to 5, characterized in that the frusto-conical braid (5) is provided with an axial slit (6).

7. Shaft part according to one of the claims 1 to 6, characterized in that the fibers (2,3) are carbon fibers, preferably from Pyrocarbon.

8. Shaft part according to one of the claims 1 to 7, characterized in that the annular structure (7) is open throughout.

9. Shaft part according to one of the claims 1 to 8, characterized in that the frusto-conical braid (5) assumes a nearly circular cross-section in the distal zone and a nearly elliptical cross-section in the proximal zone.

10. Shaft part according to one of the claims 1 to 9, characterized in that the outer contours of the braid (5) are concave when seen in longitudinal section.

## Revendications

1. Elément de broche implantable dans un os long (1), en vue d'une prothèse articulaire ou en tant que remplacement de la tige de l'os, comprenant en substance un treillis (5) en forme de tronc de cône creux et de longueur et de diamètre variables, qui est constitué de deux séries de fibres (2, 3) croisées l'une par rapport à l'autre, caractérisé en ce que le treillis (5) en forme de tronc de cône:
(a) présente dans sa zone proximale une structure annulaire (7) à laquelle les extrémités proximales des fibres (2, 3) sont fixées et
(b) présente dans sa zone distale une structure annulaire (8) à laquelle sont fixées les extrémités distales des fibres (2, 3).

2. Elément de broche selon la revendication 1, caractérisé en ce que les fibres forment un angle α avec l'axe longitudinal (4) du tronc de cône formé par le treillis (2, 3).

3. Elément de broche selon la revendication 2, caractérisé en ce que l'angle (α) des deux séries de fibres (2, 3) est approximativement de même valeur.

4. Elément de broche selon l'une des revendications 1 à 3, caractérisé en ce que l'angle α varie le long des fibres (2, 3) et de préférence diminue depuis l'extrémité distale vers l'extrémité proximale.

5. Elément de broche selon l'une des revendications 1 à 4, caractérisé en ce que les interstices (17) du treillis (5) en forme de tronc de cône sont remplis d'un matériau élastomère (9), de préférence un caoutchouc silicone ou silastique.

6. Elément de broche selon l'une des revendications 1 à 5, caractérisé en ce que le treillis (5) en forme de tronc de cône présente une fente (6) s'étendant axialement.

7. Elément de broche selon l'une des revendications 1 à 6, caractérisé en ce que les fibres (2, 3) sont des fibres de carbone, de préférence en pyrocarbone.

8. Elément de broche selon l'une des revendications 1 à 7, caractérisé en ce que la structure annulaire (7) est configurée ouverte de bout en bout.

9. Elément de broche selon l'une des revendications 1 à 8, caractérisé en ce que le treillis (5) en forme de tronc de cône présente dans sa zone distale une section transversale de la forme approximative d'un cercle et dans sa zone proximale une section transversale de la forme approximative d'une ellipse.

10. Elément de broche selon l'une des revendications 1 à 9, caractérisé en ce que les contours extérieures du treillis (5) sont configurés de forme concave en coupe longitudinale.
